# EUROPEAN PATENT APPLICATION

(11) **EP 4 509 036 A1**
(43) Date of publication of application: **19.02.2025**
(21) Application number: 24191713.7
(22) Date of filing: 30.07.2024
(51) Int. Cl.: A61B 3/117

(54) **SYSTEM AND METHOD FOR IMPROVING SCLERAL SPUR VISIBILITY IN ANTERIOR SEGMENT OCT IMAGES**

(30) Priority: 04.08.2023 EP 23189730
(71) Applicant: Optos PLC, Dunfermline, Scotland KY11 8GR (GB)
(72) Inventor: ANDERSON, Alan, Dunfermline, Scotland, KY11 8GR (GB); MUYO, Gonzalo, Dunfermline, Scotland, KY11 8GR (GB); PRECIADO, Miguel, Dunfermline, Scotland, KY11 8GR (GB); BALAGOPAL, Bavishna, Dunfermline, Scotland, KY11 8GR (GB)
(74) Representative: Hoffmann Eitle

(57) **Abstract**

A system arranged to process an anterior-segment optical coherence tomography, AS-OCT, image comprising representations of portions of a scleral spur of an eye to obtain a geometric measurement of the eye, comprising data processing hardware arranged to: process the AS-OCT image to acquire respective locations in the AS-OCT image of the representations of the portions of the scleral spur of the eye in the AS-OCT image; and obtain the geometric measurement based on the acquired locations. The system further comprises an OCT imaging system which is operable to acquire the AS-OCT image and comprises a fixation target to fix a gaze direction of the eye during acquisition of the AS-OCT image such that an axis in the AS-OCT image corresponding to a pupillary axis of the eye is aligned with a direction in the AS-OCT image corresponding to an axial imaging direction of the OCT imaging system.

## Description

### [Field]

Example aspects herein generally relate to the field of optical coherence tomography (OCT) imaging systems and, in particular, to OCT imaging systems for acquiring OCT images of at least a portion of an anterior chamber of an eye.

### [Background]

Optical coherence tomography (OCT) is an imaging technique based on low-coherence interferometry, which is widely used to acquire high-resolution two- and three-dimensional images of optical scattering media, such as biological tissue.

As is well-known, OCT imaging systems can be classified as being time-domain OCT (TD-OCT) or Fourier-domain OCT (FD-OCT) (also referred to as frequency-domain OCT), depending on how depth ranging is achieved. In TD-OCT, an optical path length of a reference arm of the imaging system's interferometer is varied in time during the acquisition of a reflectivity profile of the scattering medium being imaged by the OCT imaging system (referred to herein as the "imaging target"), the reflectivity profile being commonly referred to as a "depth scan" or "axial scan" ("A-scan"). In FD-OCT, a spectral interferogram resulting from an interference between light in the reference arm and light in the sample arm of the interferometer at each A-scan location is Fourier transformed to simultaneously acquire all points along the depth of the A-scan, without requiring any variation in the optical path length of the reference arm. FD-OCT can allow much faster imaging than scanning of the sample arm mirror in the interferometer, as all the back-reflections from the sample are measured simultaneously. Two common types of FD-OCT are spectral-domain OCT (SD-OCT) and swept-source OCT (SS-OCT). In SD-OCT, a broadband light source delivers many wavelengths to the imaging target, and all wavelengths are measured simultaneously using a spectrometer as the detector. In SS-OCT (also referred to as time-encoded frequency-domain OCT), the light source is swept through a range of wavelengths, and the temporal output of the detector is converted to spectral interference.

OCT imaging systems can also be classified as being point-scan (also known as "point detection" or "scanning point"), line-scan or full-field, depending on how the imaging system is configured to acquire OCT data at locations on the imaging target. A point-scan OCT imaging system acquires OCT data by scanning a focused sample beam across the surface of the imaging target, typically along a single line (which may be straight, or alternatively curved so as to define a circle or a spiral, for example) or along a set of (usually substantially parallel) lines on the surface of the imaging target, and acquiring an axial depth profile (A-scan) for each of a plurality of points along the line(s), one single point at a time, to build up OCT data comprising a one- or two-dimensional array of A-scans representing a two-dimensional (i.e. a B-scan) or three-dimensional (i.e. a C-scan or volumetric scan) reflectance profile of the sample.

A line-scan OCT imaging system acquires OCT data by scanning a focused line of light across the surface of the imaging target. Measured reflectance from the imaging target is used to generate OCT data comprising a two-dimensional reflectance profile (i.e. a B-scan) of the sample. By scanning the focused line of light across a plurality of locations on the imaging target, OCT data comprising a three-dimensional reflectance profile (i.e. a C-scan or volumetric scan) of the sample can be obtained. Typically, the focused line of light is straight and is scanned in a direction perpendicular to it, although in some instances it may be curved with the scanning direction adjusted accordingly. A full-field OCT imaging system acquires OCT data by projecting a beam of light onto the imaging target to acquire 'en-face' B-Scans (orthogonal to the aforementioned B-scan) which may be stacked to create a three-dimensional reflectance profile (i.e. a C-scan or volumetric scan) of the sample.

Anterior Segment OCT (AS-OCT) imaging can be performed using one of the various types of OCT imaging system summarised above to acquire a tomographic image (usually a B-scan or a C-scan) of at least a portion the anterior segment of the eye. AS-OCT imaging is performed for various reasons relating to eye health, for example for glaucoma assessment and cataract surgery (e.g. for biometry and pachymetry). A key aspect for glaucoma assessment is to determine the drainage capability at the cornea/sclera/iris junction, requiring good visibility of this region. Although it is possible to perform scans that are limited to this region (typically using specialised lens adapters), it is preferable for efficiency of data capture and storage to capture a full anterior chamber OCT image (also widely referred to as "full-chamber" or "full anterior segment" OCT image), which encompasses the whole anterior chamber of the eye and the surrounding portions of the anterior segment of the eye, typically including the cornea, sclera, iris, anterior of the crystalline lens and scleral spur.

AS-OCT imaging may be performed not only for glaucoma assessment but for a variety of other purposes, wherein geometric measurements of the eye are made. Such geometric measurements may include a measurement of a distance within the eye, such as the anterior chamber width (ACW, also known as spur-to-spur or angle-to-angle distance), the lens vault (LV), the angle opening distance (AOD, e.g. the AOD500) or anterior chamber depth (ACD) of the eye, or a measurement of an angle within the eye, such as the anterior chamber angle (ACA), the trabecular-iris angle (TIA) or the iris-lens angle of the eye, for example, and/or related areas such as the trabecular-iris space area (TISA; e.g. the TISA500), for example. The geometric measurements often rely on an accurate identification of the location of one or more portions of the scleral spur of the eye within an AS-OCT image (the scleral spur being in the angle region of the anterior segment, where the iris meets the sclera), which can serve as useful landmarks or points of reference. The better the visibility of the scleral spur landmark, the more accurate measurements based thereon can be. However, in an AS-OCT image in the form of an AS-OCT B-scan, for example, the visibility or contrast of parts of the scleral spur that oppose each other in the AS-OCT B-scan (i.e. representations of the scleral spur that appear on opposite sides of the anterior segment imaged in the B-scan) tends to differ. It is consequently often difficult to accurately locate at least one of the parts of the scleral spur, and therefore to obtain an accurate geometric measurement from the image. It is therefore highly desirable to allow the scleral spur in an AS-OCT image (e.g. a full-chamber OCT image) to be located more accurately.

### [Summary]

There is provided, in accordance with a first example aspect herein, a system arranged to process an anterior-segment optical coherence tomography, AS-OCT, image comprising representations of portions of a scleral spur of an eye to obtain a geometric measurement of the eye. The system comprises data processing hardware arranged to: process the AS-OCT image to acquire respective locations in the AS-OCT image of the representations of the portions of the scleral spur of the eye in the AS-OCT image; and obtain the geometric measurement based on the acquired locations. The system further comprises an OCT imaging system operable to acquire the AS-OCT image, the OCT imaging system comprising a fixation target arranged to fix a gaze direction of the eye during acquisition of the AS-OCT image such that an axis in the AS-OCT image corresponding to a pupillary axis of the eye is aligned with a direction in the AS-OCT image corresponding to an axial imaging direction of the OCT imaging system. The fixation target may be arranged to fix the gaze direction of the eye during acquisition of the AS-OCT image such that the pupillary axis of the eye, rather than a visual axis of the eye, is aligned with the axial imaging direction of the OCT imaging system, with the axis in the AS-OCT image corresponding to the pupillary axis of the eye (rather than an axis in the AS-OCT image corresponding to the visual axis of the eye) consequently being aligned with the direction in the AS-OCT image corresponding to the axial imaging direction of the OCT imaging system. Accordingly, the fixation target may be arranged to fix the gaze direction of the eye during acquisition of the AS-OCT image such that the pupillary axis of the eye is more closely aligned with the axial imaging direction of the OCT imaging system than is the visual axis of the eye.

There is provided, in accordance with a second example aspect herein, the use of a fixation target of an OCT imaging system to equalise image contrast in representations of portions of a scleral spur of an eye in an anterior-segment OCT, AS-OCT, image acquired by the OCT imaging system, wherein the fixation target is used to equalise the image contrast by fixing a gaze direction of the eye during acquisition of the AS-OCT image such that an axis in the AS-OCT image corresponding to a pupillary axis of the eye is aligned with a direction in the AS-OCT image corresponding to an axial imaging direction of the OCT imaging system. The fixation target may be used to equalise the image contrast by fixing the gaze direction of the eye during acquisition of the AS-OCT image such that the pupillary axis of the eye, rather than a visual axis of the eye, is aligned with the axial imaging direction of the OCT imaging system, with the axis in the AS-OCT image corresponding to the pupillary axis of the eye (rather than an axis in the AS-OCT image corresponding to the visual axis of the eye) consequently being aligned with the direction in the AS-OCT image corresponding to the axial imaging direction of the OCT imaging system. Accordingly, the fixation target may fix the gaze direction of the eye during acquisition of the AS-OCT image such that the pupillary axis of the eye is more closely aligned with the axial imaging direction of the OCT imaging system than is the visual axis of the eye.

There is provided, in accordance with a third example aspect herein, a method of processing an anterior-segment optical coherence tomography, AS-OCT, image acquired by an OCT imaging system, which comprises representations of portions of a scleral spur of an eye, to obtain a geometric measurement based on one or more anatomical features in the AS-OCT image. The method comprises: acquiring the AS-OCT image whilst a gaze direction of the eye is fixed by the eye fixating on a fixation target such that an axis in the AS-OCT image corresponding to a pupillary axis of the eye is aligned with a direction in the AS-OCT image corresponding to an axial imaging direction of the OCT imaging system; processing the acquired AS-OCT image to acquire respective locations in the AS-OCT image of the representations of the portions of the scleral spur of the eye in the AS-OCT image; and obtaining the geometric measurement based on the acquired locations in the AS-OCT image. The AS-OCT image may be acquired whilst the gaze direction of the eye is fixed by the eye fixating on the fixation target such that the pupillary axis of the eye, rather than a visual axis of the eye, is aligned with the axial imaging direction of the OCT imaging system, with the axis in the AS-OCT image corresponding to the pupillary axis of the eye (rather than an axis in the AS-OCT image corresponding to the visual axis of the eye) consequently being aligned with the direction in the AS-OCT image corresponding to the axial imaging direction of the OCT imaging system. Accordingly, the AS-OCT image may be acquired whilst the gaze direction of the eye is fixed by the eye fixating on the fixation target such that the pupillary axis of the eye is more closely aligned with the axial imaging direction of the OCT imaging system than is the visual axis of the eye.

### [Brief Description of the Drawings]

Example embodiments will now be described in detail, by way of non-limiting example only, with reference to the accompanying figures described below. Like reference numerals appearing in different ones of the figures can denote identical or functionally similar elements, unless indicated otherwise.
Figure 1 is a schematic illustration of a system 100 according to a first example embodiment herein.
Figure 2 is a schematic illustration of a B-scan 200 acquired by an OCT imaging system 110 of the system 100 according to the first example embodiment herein.
Figure 3 is a schematic illustration of a front view of a display device 300 of the system 100, which displays a graphic 310 as an example of a fixation target for fixing a gaze direction of an eye.
Figure 4 is a schematic illustration of a programmable signal processing hardware, which may be configured to perform the functions of data processing hardware 150 and/or controller 115 of the OCT imaging system 110 described herein.
Figure 5 is a flow diagram illustrating a process by which an AS-OCT image 130 acquired by the OCT imaging system 110 is processed by data processing hardware 150 of the system 100 to obtain a geometric measurement of the eye according to the first example embodiment herein.
Figure 6 is a flow diagram illustrating a process by which the controller 115 of the OCT imaging system 110 controls the fixation target 120 to fix the gaze direction of the eye 140 during acquisition of the AS-OCT image 130 in the first example embodiment herein.
Figure 7 is a flow diagram illustrating an alternative process by which the controller 115 of the OCT imaging system 110 may acquire an indication, ({Ø}*_{N}*)_{*k*,} of an orientation of a pupillary axis 153 of the eye relative to a visual axis of the eye in a first alternative implementation of the first example embodiment herein.
Figure 8 is a schematic illustration of an example calibration B-scan 800 acquired by the OCT imaging system 110 in the first alternative implementation of the first example embodiment herein.
Figure 9 is a flow diagram of a process by which the controller 115 of the OCT imaging system 110 can determine the orientation of an axis in a calibration B-scan corresponding to the pupillary axis of the eye relative to a direction in the calibration B-scan corresponding to the axial imaging direction of the OCT imaging system 110 in the first alternative implementation of the first example embodiment herein.
Figure 10 is a flow diagram illustrating a second alternative process by which the controller 115 of the OCT imaging system 110 may acquire the indication, ({Ø}*_{N}*)*ₖ*, of an orientation of the pupillary axis of the eye relative to the visual axis of the eye in a second alternative implementation of the first example embodiment herein.
Figures 11A and 11B are schematic illustrations of an example first calibration B-scan 1100 and an example calibration second B-scan 1140, respectively, according to the second alternative implementation of the first example embodiment herein.
Figures 12A and 12B are an uncorrected B-scan 1200 and a B-scan 1250 corrected according to the first example embodiment herein, respectively.

### [Detailed Description of Example Embodiments]

The inventors have recognised that, due to the non-alignment of the visual axis and the pupillary axis of the eye, conventional OCT imaging systems for AS-OCT imaging (e.g. for full-chamber imaging) tend to acquire AS-OCT B-scans in which anatomical features, such as the iris, appear with a small degree of tilt relative to the lateral direction in the B-scan, i.e. perpendicular to the axial direction in the B-scan (the axial direction being a direction in the B-scan along which elements of each component A-scan of the B-scan are arrayed). This tilt is generally seen as a nuisance, and it is typically compensated for in post-processing to provide the clinician with a symmetric B-scan.

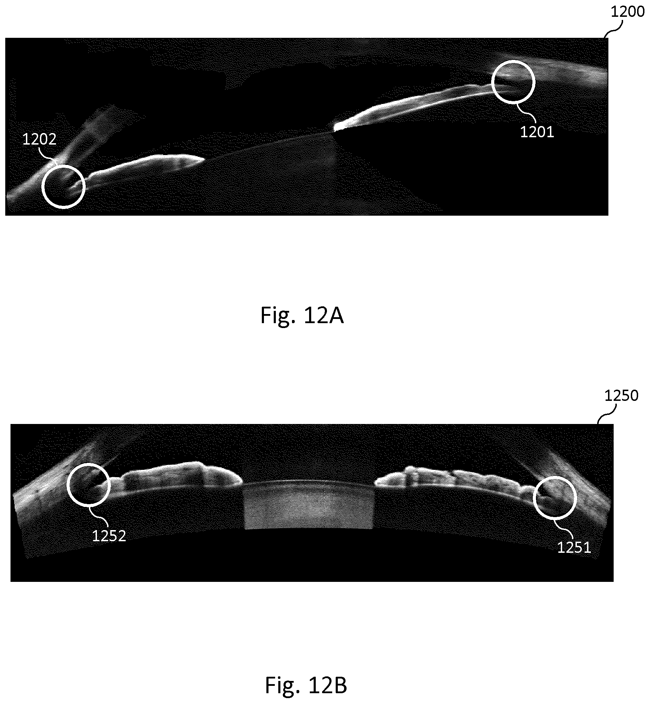

On closer inspection of AS-OCT B-scans, the inventors have found that the degree of tilt observed in acquired B-scans is correlated with the visibility and contrast of representations of the opposing portions of the scleral spur in the B-scans (i.e. the different sectional views of the scleral spur that appear on opposite sides of the B-scan, as shown at 1201 and 1202 in Figure 12A, for example). More particularly, the inventors have noticed that the difference between the respective visibilities (or contrasts) of the representations of the two opposing portions of the scleral spur in the B-scan tends to increase with increasing degree of tilt of the anatomical features of the anterior segment relative to the frame of the B-scan.

The inventors have further recognised that the overall visibility or contrast of the representations of the portions of the scleral spur in an AS-OCT B-scan, and therefore the accuracy of measurements that are made using these landmarks, can be improved by removing or at least reducing the tilt in the B-scan at its source, specifically by modifying the acquisition of the B-scan so that the pupillary axis of the eye (rather than the visual axis) is aligned with the axial imaging direction of the OCT imaging system. More specifically, the OCT imaging system may be modified to comprise a fixation target that is arranged to fix a gaze direction of the eye during acquisition of the B-scan such that an axis in the B-scan corresponding to a pupillary axis of the eye is aligned with a direction in the B-scan corresponding to an axial imaging direction of the OCT imaging system. This may not only remove or reduce the degree of tilt in the B-scan but also tends to equalise the visibility or contrast of the representations of the scleral spur in the B-scan, allowing more accurate geometrical measurements of the anterior segment of the eye to be made. It will be appreciated that, although the foregoing discussion has been framed in the context of B-scans, the described principles are generally applicable to anterior segment OCT (AS-OCT) images in the form of C-scans as well.

Example embodiments will now be described in detail with reference to accompanying drawings.

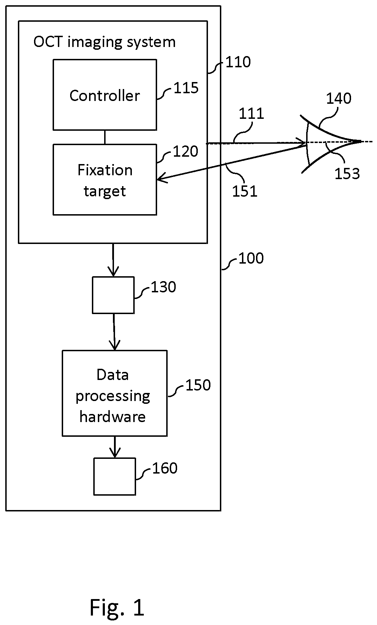

Figure 1 is a schematic illustration of a system 100 according to a first example embodiment herein, which is arranged to process an AS-OCT image to obtain a geometric measurement of an eye.

The system 100 comprises an OCT imaging system 110, which is controlled by a controller 115 thereof and comprises a fixation target 120. The OCT imaging system 110 is arranged to acquire an AS-OCT image 130 of an eye 140 which comprises representations of (opposing) portions of a scleral spur of the eye 140 (e.g. portions of the scleral spur which are at opposing sides of the anterior chamber of the eye 140). The AS-OCT image 130 of the eye 140 may be a full-chamber AS-OCT image which encompasses a whole anterior chamber of an eye 140 and surrounding portions of an anterior segment of the eye 140, including the (opposing) portions of a scleral spur of the eye 140. During acquisition of the AS-OCT image 130, the fixation target 120 is arranged to fix the gaze direction 151 of the eye 140 such that an axis in the AS-OCT image 130 corresponding to a pupillary axis 153 of the eye 140 is aligned with a direction in the AS-OCT image corresponding to an axial imaging direction 111 of the OCT imaging system 110.

The system 100 further comprises data processing hardware 150, which is arranged to process the AS-OCT image 130 to obtain a geometric measurement 160 of the eye 140. The geometric measurement 160 of the eye 140 is obtained by the data processing hardware 150 based on locations of the representations of the (opposing) portions of the scleral spur of the eye 140 in the AS-OCT image 130 and, depending on the selected geometric measurement 160, representations of one or more other anatomical features of the eye 140 in the AS-OCT image 130, as described in more detail below. Although the data processing hardware 150 may be provided as a component that is separate from the OCT imaging system 110, as in the present example embodiment, the data processing hardware 150 may alternatively form part of the OCT imaging system 110, with its functions performed by the controller 115, for example.

The OCT imaging system 110 may, as in the present example embodiment, be a point-scan Fourier-domain OCT imaging system. However, the form of the OCT imaging system 110 is not so limited, and may alternatively take the form of a time-domain system, and may furthermore alternatively be line-scan or a full-field OCT imaging system. Further, the OCT imaging system 110 may be operable to acquire both AS-OCT images and posterior-segment OCT (PS-OCT) images of the eye 140.

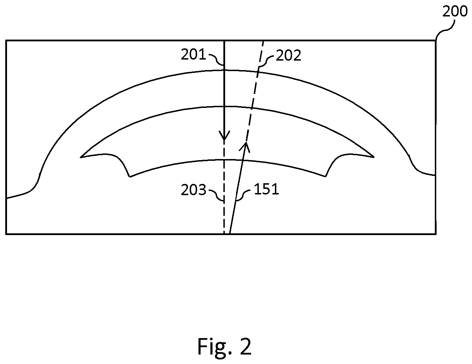

Figure 2 is a schematic illustration of an AS-OCT image 130 in the exemplary form of a full-chamber B-scan 200 representing a cross-section of the whole anterior chamber of the eye 140 and the surrounding portions of the anterior segment of the eye 140, including opposing portions of a scleral spur of the eye 140. The B-scan 200 comprises a direction 201 corresponding to an axial imaging direction 111 of the OCT imaging system 110, an axis 203 corresponding to a pupillary axis 153 of the eye 140 and a second axis 202 corresponding to the visual axis of the eye 140, which defines the gaze direction 151 of the eye 140. However, the AS-OCT image 130 may more generally be a B-scan which does not encompass the whole anterior chamber of the eye 140 but nevertheless comprises the representations of the (opposing) portions of the scleral spur of the eye 140 (e.g. a B-scan covering a section of the anterior chamber of the eye 140 along the axial direction which comprises the portions of the scleral spur of the eye 140). The AS-OCT image 130 may alternatively be a C-scan comprising the representations of the (opposing) portions of the scleral spur of the eye 140, which may encompass the whole anterior chamber of the eye 140 and the surrounding portions of the anterior segment of the eye 140, as described below.

The fixation target 120 is arranged to fix the gaze direction 151 of the eye 140 (i.e. a direction away from the eye 140, along the visual axis of the eye 140) during acquisition of the AS-OCT image 130 such that the axis 203 in the AS-OCT image 130 corresponding to the pupillary axis 153 of the eye 140 is aligned with the direction 201 in the AS-OCT image 130 corresponding to the axial imaging direction 111 of the OCT imaging system 110. That is, the fixation target 120 is arranged such that, when the eye 140 fixates on it so as to fix a gaze direction 151 of the eye 140 during acquisition of the AS-OCT image (130), the axis 203 within the AS-OCT image 130 that is representative of the pupillary axis 153 of the eye 140 is parallel to the direction 201 in the AS-OCT image 130 that is representative of the axial imaging direction 111 of the OCT imaging system 110. The axis 203 may, more generally, be aligned with the direction 201 to within a predetermined tolerance (e.g. 0.01, 0.1 or 1 degrees). This is shown in Figure 2, where the arrangement of the fixation target 120 has fixed the gaze direction 151 of the eye 140 during acquisition of the B-scan 200 such that the axis 203 in the B-scan 200 corresponding to pupillary axis 153 of the eye 140 is parallel in the B-scan 200 with the direction 201 corresponding to the axial imaging direction 111 of the OCT imaging system 110.

The position of the fixation target 120 relative to the eye 140 may, as in the present example embodiment, be controllable by the OCT imaging system 110 (specifically by the controller 115 thereof) so as to control a gaze direction 151 of the eye 140 when the eye 140 fixates on the fixation target 120. The process by which the OCT imaging system 110 sets the fixation target 120 to fix the gaze direction 151 of the eye 140 during acquisition of the AS-OCT image 130 such that the axis 203 in the AS-OCT image 130 corresponding to the pupillary axis 153 of the eye 140 is aligned with the direction 201 in the AS-OCT image 130 corresponding to the axial imaging direction 111 of the OCT imaging system 110 is described in more detail below. However, the position of the fixation target 120 may alternatively be set relative to an expected position of the eye 140 (i.e. a location set by the position of a chin rest or the like, where the eye 140 is located relative to the OCT imaging system 110 for the OCT imaging system 110 to acquire the AS-OCT image 130) during manufacture such that, during acquisition of the AS-OCT image 130, the axis 203 in the AS-OCT image 130 corresponding to the pupillary axis 153 of the eye 140 is aligned with the direction 201 in the AS-OCT image 130 corresponding to the axial imaging direction 111 of the OCT imaging system 110.

The fixation target 120 may, as in the present example embodiment, be integrated into the sample arm of the OCT imaging system 110 by coupling light from the fixation target 120 into the optical pathway of the sample arm using a beam splitter/combiner. Where the OCT imaging system 110 is a line-scan or point-scan OCT imaging system, the beam splitter/combiner may be placed between the scanning mirror(s) and the eye 140 or before at least one of the scanning mirrors in the optical path towards the eye 140. Where the fixation target 120 is placed before at least one of the scanning mirrors in the optical path towards the eye 140, the light from the fixation target 120 may be pulsed to coincide with predefined position(s) of the at least one of the scanning mirrors to ensure that the position of the fixation target 120 from the perspective of the eye 140 remains fixed as the at least one of the scanning mirrors move.

The OCT imaging system 110 may set the position of the fixation target 120 to achieve the alignment between direction 201 and axis 203 described above using techniques well-known to those skilled in the art. For example, the position of the fixation target 120 relative to the eye 140 may be set to achieve the alignment between direction 201 and axis 203 described above based on a ray tracing model of the eye and of the optics of the OCT imaging system 110 between the fixation target 120 and the eye 140. Alternatively, the position of the fixation target 120 relative to the eye 140 required to achieve the alignment between direction 201 and axis 203 described above may be estimated based on trigonometry, using the optical path length, L, from a point within the eye 140 (e.g. the centre of the pupil or geometric centre of the eyeball) to the fixation target 120.

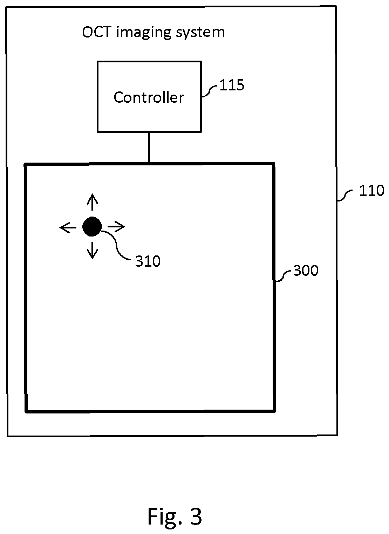

Figure 3 is a schematic illustration of an example implementation of the OCT imaging system 110, wherein the fixation target 120 takes the form of a graphic 310 which is displayed by a display device 300 forming part of the OCT imaging system 110. The display position of the graphic 310 relative to the eye 140 is controllable by the controller 115 so as to control a gaze direction 151 of the eye 140 when the eye 140 fixates on the graphic 310. In other words, the position of the eye 140 relative to the OCT imaging system 110 is fixed (e.g. by placing the patient's chin on a chin rest of the OCT imaging system 110) and the display position of the graphic 310 is moved around the displayable area of the display device 300, as indicated by the arrows in Figure 3, so to move (steer) the gaze direction 151 of the eye 140 when the eye 140 fixates on the graphic 310. Although the graphic 310 takes the form of a dot in Figure 3, it will be appreciated that the form of the graphic is not so limited, and may alternatively be provides as a cross, circle or any shape onto which the eye 140 can fixate. Furthermore, the fixation target 120 may be implemented in a form other than a display device. For example, the fixation target 120 may include a light source (e.g. a light-emitting diode) attached to an actuator that is controllable by the controller 115 to move the light source relative to the eye 140 so as to control a gaze direction 151 of the eye 140 when the eye 140 fixates on the light source.

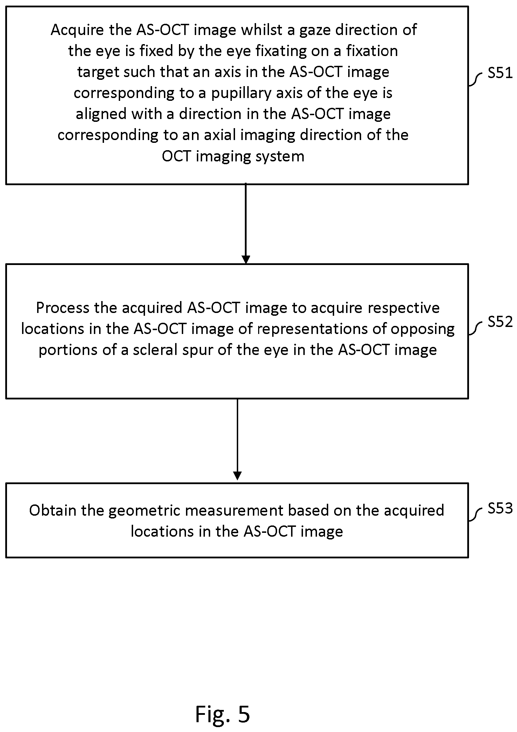

The data processing hardware 150 is arranged to process the AS-OCT image 130 to acquire respective locations in the AS-OCT image 130 of the representations of the (opposing) portions of the scleral spur of the eye 140 in the AS-OCT image 130, the details of which are described below with reference to process S52 of Figure 5. The data processing hardware 150 is further arranged to obtain (or determine) the geometric measurement 160 based on the acquired locations, as described below with reference to process S53 of Figure 5.

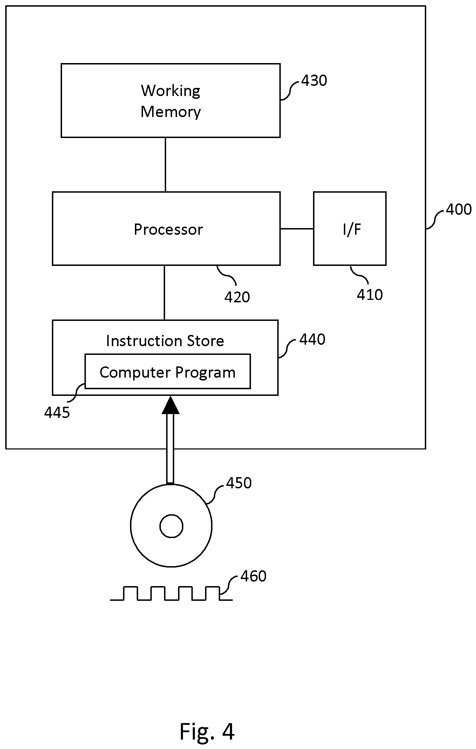

The data processing hardware 150 and the controller 115 of the OCT imaging system 110 may be provided in any suitable form, for example as a programmable signal processing hardware 400 of the kind illustrated schematically in Figure 4.

The programmable signal processing apparatus 400 comprises a communication interface (I/F) 410 for communication data externally. Where the apparatus 400 implements the data processing hardware 150, the I/F 410 may be arranged to receive the AS-OCT image 130 from the OCT imaging system 110 and output the geometric measurement 160 and/or a graphical representation thereof (for example, as overlaid on the AS-OCT image 130) for displaying on a display, such a computer screen or the like. Where the apparatus 400 implements the controller 115 of the OCT imaging system 110, the I/F 410 may be arranged to receive instructions for acquiring the AS-OCT image 130 (e.g. a target location and a command to initiate the acquisition procedure), and to output the AS-OCT image 130.

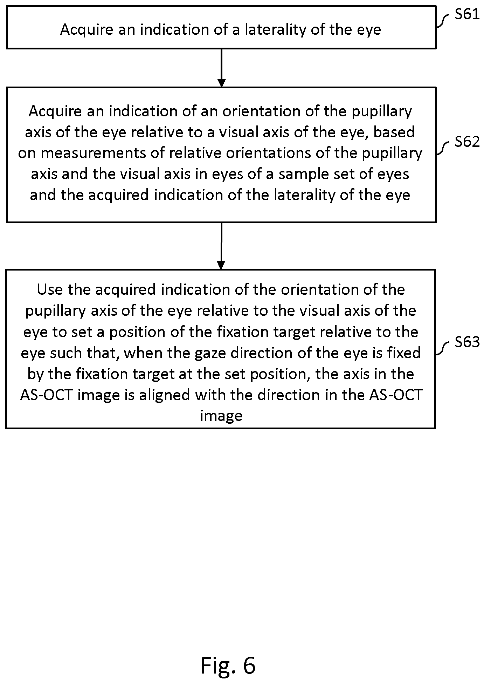

The signal processing hardware 400 further comprises a processor (e.g. a Central Processing Unit, CPU, and/or a Graphics Processing Unit, GPU) 420, a working memory 430 (e.g. a random-access memory) and an instruction store 440 storing a computer program 445 comprising the computer-readable instructions which, when executed by the processor 420, cause the processor 420 to perform various functions including those of the data processing hardware 150 and/or the controller 115 of the OCT imaging system 110 described herein. The working memory 430 stores information used by the processor 420 during execution of the computer program 445. The instruction store 440 may comprise a ROM (e.g. in the form of an electrically erasable programmable read-only memory (EEPROM) or flash memory) which is pre-loaded with the computer-readable instructions. Alternatively, the instruction store 440 may comprise a RAM or similar type of memory, and the computer-readable instructions of the computer program 445 can be input thereto from a computer program product, such as a non-transitory, computer-readable storage medium 450 in the form of a CD-ROM, DVDROM, etc. or a computer-readable signal 460 carrying the computer-readable instructions. In any case, the computer program 445, when executed by the processor 420, causes the processor 420 to perform the functions of the data processing hardware 150 and/or controller 115 of the OCT imaging system 110 as described herein. In other words, the data processing hardware 150 of the first example embodiment may comprise a computer processor 420 and a memory 440 storing computer-readable instructions which, when executed by the computer processor 420, cause the computer processor 420 to process the AS-OCT image 130 to acquire respective locations in the AS-OCT image 130 of representations of (opposing) portions of the scleral spur of the eye 140 in the AS-OCT image 130 and obtain the geometric measurement 160 based on the acquired locations. Similarly, the controller 115 of the OCT imaging system 110 of the first example embodiment may comprise a computer processor 420 and a memory 440 storing computer-readable instructions which, when executed by the computer processor 420, cause the computer processor 420 to perform the processes described below in the present example embodiment (including the process in S51 of Figure 5 and the processes in Figures 6, 7, 9 and 10).

It should be noted that the functions of both the data processing hardware 150 and the controller 115 of the OCT imaging system 110 may be performed by the programmable signal processing hardware 400. Further, the data processing hardware 150 and/or the controller 115 of the OCT imaging system 110 may alternatively be implemented in non-programmable hardware, such as an ASIC, an FPGA or other integrated circuit dedicated to performing the described functions of the data processing hardware 150 and/or the controller 115 of the OCT imaging system 110 (as the case may be), or a combination of such non-programmable hardware and programmable hardware as described above with reference to Figure 4.

Figure 5 is a flow diagram illustrating a process by which the AS-OCT image 130 acquired by the OCT imaging system 110 may, as in the present example embodiment, be processed by the data processing hardware 150 to obtain a geometric measurement 160 of the eye 140.

In process S51 of Figure 5, the OCT imaging system 110 acquires the AS-OCT image 130 whilst the gaze direction 151 of the eye 140 is fixed by the eye 140 fixating on the fixation target 120 such that the axis 203 in the AS-OCT image 130 corresponding to the pupillary axis 153 of the eye 140 is aligned with the direction 201 in the AS-OCT image 130 corresponding to the axial imaging direction 111 of the OCT imaging system 110. The process by which the controller 115 of the OCT imaging system 110 sets the fixation target 120 to fix the gaze direction 151 of the eye 140 in this way is described in more detail below. However, as noted previously, the fixation target 120 may alternatively be set to fix the direction 151 of the eye 140 in the aforementioned manner during the manufacture of the OCT imaging system 110.

In process S52 of Figure 5, the data processing hardware 150 processes the acquired AS-OCT image 130 to acquire respective locations in the AS-OCT image 130 of the representations of the (opposing) portions of the scleral spur of the eye 140 in the AS-OCT image 130. This may be achieved using any of the techniques well-known to those skilled in the art. For example, the data processing hardware 150 may employ deep learning methods, which make use of trainable, multilayer neural networks, to identify the locations of the (opposing) portions of the scleral spur of the eye 140 in the B-scan 200. As another example, the data processing hardware 150 may process the acquired AS-OCT image 130 by causing it to be displayed to the user on a display screen, and acquire the respective locations in the AS-OCT image 130 of the representations of the (opposing) portions of the scleral spur of the eye 140 in the AS-OCT image 130 by receiving designations of these locations that have been input by the user (using a mouse and/or keyboard, or via one or more touch interactions in case the display screen is a touch-screen, for example) after having inspected the displayed AS-OCT image 130. Further details of example techniques known to those skilled in art are provided in Benjamin Y.Xu et al., "Deep Neural Network for Scleral Spur Detection in Anterior Segment OCT Images: The Chinese American Eye Study", Transl Vis Sci Technol., 30 March 2020, which is hereby incorporated by reference in its entirety.

In process S53 of Figure 5, the data processing hardware 150 obtains (or determines) the geometric measurement 160 based on the acquired locations. This may be achieved using techniques well-known to those skilled in the art. For example, the data processing hardware 150 may determine the ACW of the eye 140 as the distance between the acquired locations of the representations of the portions of the scleral spur in the B-scan 200. As a further example, the data processing hardware 150 may determine any of the AOD, TIA, TISA and the ACA of the eye 140 based on the acquired locations of the representations of the portions of the scleral spur in the B-scan 200 and on the boundaries of the anterior chamber in the B-scan 200 (e.g. the anterior iris surface and the posterior corneal surface). The boundaries of the anterior chamber in the B-scan 200 may be determined by the data processing hardware 150 by using anterior chamber segmentation techniques well-known to those skilled in the art, such as anterior chamber segmentation employing deep learning methods. Further details regarding the determining of anterior chamber angle parameters may be found in Guangqian Yang et al., "Automatic measurement of anterior chamber angle parameters in AS-OCT images using deep learning," Biomed. Opt. Express 14, 1378-1392 (2023), which is hereby incorporated by reference in its entirety.

Figure 6 is a flow diagram illustrating a process by which the controller 115 of the OCT imaging system 110 may, as in the present example embodiment, set the fixation target 120 to fix the gaze direction 151 of the eye 140 during acquisition of the AS-OCT image 130 in step S51 of Figure 5.

In optional process S61 of Figure 6, the controller 115 acquires the laterality of the eye 140 (i.e. whether the eye 140 is the right eye or the left eye of a subject). The controller 115 may acquire the indication of the laterality of the eye 140 by receiving an input from a clinician operating the OCT imaging system 110, for example. However, the indication of the laterality of the eye 140 may alternatively be automatically determined by the controller 115 using techniques known to those skilled in the art. As noted above, process S61 is optional and may not be performed, as described below in relation to alternative implementations of the first example embodiment.

In process S62 of Figure 6, the controller 115 acquires an indication, *I*, of an orientation (e.g. as defined by an angle, for example as in a polar coordinate system, or as defined by two angles, for example as in a spherical coordinate system) of the pupillary axis 153 of the eye 140 relative to the visual axis 152 of the eye 140 based on measurements of relative orientations of the pupillary axis and the visual axis in eyes of a sample set of eyes and the acquired laterality of the eye 140. The controller 115 may acquire the indication of the orientation of the pupillary axis 153 of the eye 140 relative to the visual axis 152 of the eye from data from a memory (e.g. the memory 440 of Figure 4) of the OCT imaging system 110. However, the indication, *I*, of the orientation may alternatively be acquired in other ways, for example by downloading the indication, *I*, from a remote server or by retrieving measurements of relative orientations of the pupillary axis and the visual axis in eyes of the sample set of eyes corresponding to the acquired laterality of the eye 140 from a remote server and calculating, as the indication, *I*, of the orientation, a representative value (e.g. a mean or a median) of the retrieved measurements.

The measurements of relative orientations of the pupillary axis and the visual axis in eyes of the sample set of eyes (i.e. as sampled from a population) may, as in the present example embodiment, comprise respective first values, *(*Ø*_{NT},* Ø*_{SI}*)*_{R}*, indicative of first components of the kappa angle (i.e. the angle between the pupillary axis and the visual axis in an eye) of right eyes in the nasal-temporal direction (e.g. with the positive angle direction from the nasal side to the temporal side of an eye) and of second components of the kappa angle of right eyes in the superior-inferior direction (e.g. with the positive angle direction from the superior side to the inferior side of an eye), and further comprise respective second values, (Ø*_{NT}*, Ø*_{SI}*)*_{L}* indicative of the first components of the kappa angle of left eyes in the nasal-temporal direction and of second components of the kappa angle of left eyes in the superior-inferior direction. Accordingly the memory accessed by the controller 115 may store a representative value (e.g. a mean or a median) of the first values, (Ø*_{NT}, Ø_{SI}*)*_{R,}* and a representative value of the second values, (Ø*_{NT},* Ø*_{SI})_{L},* from which the controller 115 may acquire the indication of the orientation, *(*Ø*_{NT}, Ø_{SI}*)*ₖ,* as the representative value which corresponds to the indication of the laterality of the eye 140. However, the memory of the OCT imaging system 110 may alternatively store the first values, *(*Ø*_{NT},* Ø*_{SI})_{R}* and the second values, (Ø*_{NT},* Ø*_{SI}*)*_{L}* and the controller 115 may retrieve the set of values which corresponds to the indication of the laterality of the eye 140 and use them to calculate the representative value as the indication of the orientation, (Ø*_{NT},* Ø*_{SI}*)*ₖ.*

Further, the measurements of relative orientations of the pupillary axis and the visual axis in eyes of the sample set of eyes may take other forms. For example, under an assumption of symmetry between the right eyes and left eyes within a sample set of eyes, the measurements of the relative orientations may comprise values, (Ø*_{NT},* Ø*_{SI}*)*_{E},* indicative of first components of the kappa angle in the nasal-temporal direction and of second components of the kappa angle in the superior-inferior direction of respective eyes in the sample set of eyes. The acquired indication of the laterality of the eye 140 can therefore be used to resolve the measurements of the relative orientations in the frame of reference of the OCT imaging system 110 to acquire the indication, *I*, of the orientation, as the nasal-temporal direction is flipped between right and left eyes (i.e. the direction defining a positive angle in the right and left eyes is reversed relative to a common frame of reference).

In addition, the indication, *I*, of the orientation may approximate the orientation of the pupillary axis 153 of the eye 140 relative to the visual axis of the eye 140 in one direction. In such cases, the measurements of the relative orientations may comprise either the values (Ø*_{NT}*)*,* or the values (Ø*_{SI}*), indicative of the kappa angle in one dimension (and optionally for the right eye or left eye, or under the assumption of symmetry) either in the nasal-temporal direction or in the superior-inferior direction, respectively, as is described in more detail below. Although the relative orientations have been described above as being relative to the nasal-temporal direction and the superior-inferior direction, any two orthogonal directions (or any first direction and second direction with at least an orthogonal component to the first direction) in a plane defined by the nasal-temporal and superior-inferior directions may be used as the frame of reference.

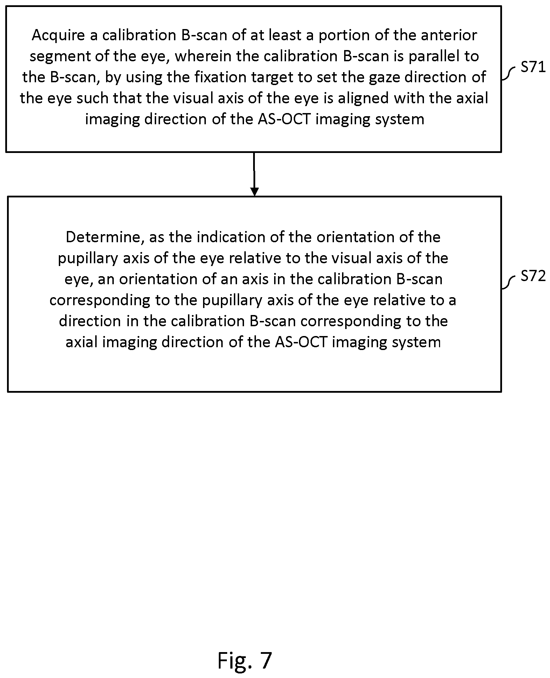

Figure 7 is a flow diagram illustrating an alternative process to process S62 in Figure 6, by which the controller 115 of the OCT imaging system 110 may acquire the indication, *I*, of the orientation of the pupillary axis 153 of the eye 140 relative to the visual axis 152 of the eye 140 in a first alternative implementation of the present example embodiment. The inventors have recognised that, although the visual axis 152 of the eye 140 may in some cases be difficult to identify within an AS-OCT image of the eye 140 based on anatomical features of the eye 140, it can be arranged to be parallel to the axial imaging direction 111 of the OCT imaging system 110 by use of the fixation target 120 to manipulate the gaze direction 151 of the eye 140, thus allowing the orientation of the pupillary axis 153 of the eye 140 (which can more easily be identified based on at least anatomical feature of the eye 130 in the AS-OCT image) to be determined relative to the assumed position of the visual axis of the eye 130. Where the alternative process described below is used, process S61 in Figure 6 may be omitted.

In process S71 of Figure 7, the controller 115 controls the OCT imaging system 110 to acquire a calibration B-scan of at least a portion of the anterior segment of the eye 140, for determining an orientation of an axis in the calibration B-scan corresponding to the pupillary axis 153 of the eye 140 relative to a direction in the calibration B-scan corresponding to the axial imaging direction 111 of the OCT imaging system 110 (e.g. by the calibration B-scan having an axis corresponding to the pupillary axis 153 of the eye 140). The controller 115 controls the OCT imaging system 110 to acquire the calibration B-scan, which is parallel to the B-scan 200, by using the fixation target 120 to set the gaze direction 151 of the eye 140 such that the visual axis 152 of the eye 140 is aligned with the axial imaging direction 111 of the OCT imaging system 110. That is, during the acquisition of the calibration B-scan, the controller 115 sets the position of the fixation target 110 relative to the eye 140 such that, when the gaze direction 151 of the eye is 140 fixed by the fixation target 120 at the set position, the gaze direction 151 of the eye 140 (along which the visual axis of the eye 140 runs) is aligned with the axial imaging direction 111 of the OCT imaging system 110. The calibration B-scan may encompass the whole anterior chamber of the eye 140 and surrounding portions of the anterior segment of the eye 140, and may include at least one portion of the scleral spur of the eye 140. Note that the calibration B-scan is acquired before the B-scan 200, so it is parallel to the expected, or predetermined, plane of the B-scan 200.

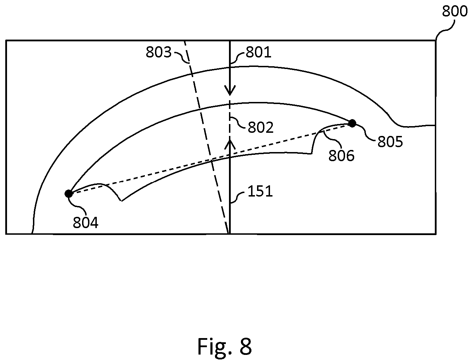

Figure 8 is a schematic illustration of an example calibration B-scan 800 acquired by the OCT imaging system 110 in the above-described manner. As shown, an axis 802 in the calibration B-scan 800 corresponding to the visual axis of the eye 140 is parallel with a direction 801 in the calibration B-scan 800 corresponding to the axial imaging direction 111 of the OCT imaging system 110, although it may, more generally, be aligned with the direction 801 to within a predetermined tolerance (e.g. 0.01, 0.1 or 1 degrees) from the direction 801.

In process S72 of Figure 7, the controller 115 determines, as the indication *I*, of the orientation of the pupillary axis 153 of the eye 140 relative to the visual axis 152 of the eye 140, an orientation of an axis in the calibration B-scan 800 corresponding to the pupillary axis 153 of the eye 140 relative to a direction in the calibration B-scan 800 corresponding to the axial imaging direction 111 of the OCT imaging system 110. As the calibration B-scan 800 is parallel to the B-scan 200, the orientation of the pupillary axis 153 of the eye 140 relative to the visual axis 152 of the eye 140 in the calibration B-scan 800 will correspond to the orientation of the pupillary axis 153 of the eye 140 relative to the visual axis 152 of the eye 140 in the B-scan 200 subsequently acquired by the OCT imaging system 110, so it can be used as the acquired indication of the orientation for the purpose of the correction applied in process S63 of Figure 6, which is described in detail below.

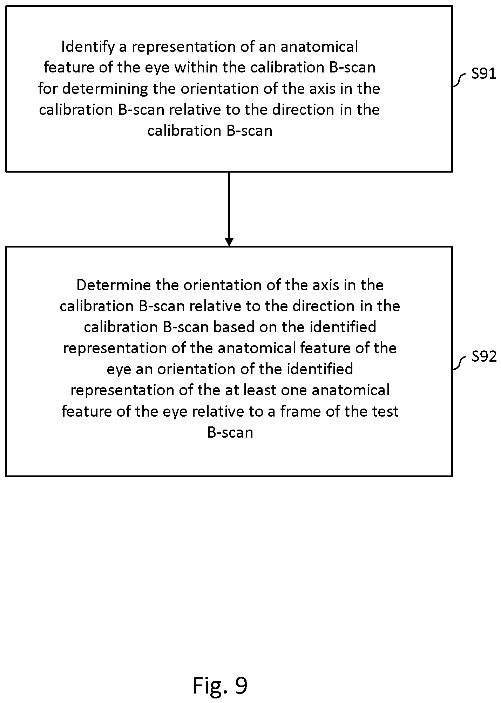

Figure 9 is a flow diagram illustrating a process by which the controller 115 of the OCT imaging system 110 may, as in the first alternative implementation of the present example embodiment, determine the orientation of the axis in the calibration B-scan 800 corresponding to the pupillary axis 153 of the eye 140 relative to the direction in the calibration B-scan 800 corresponding to the axial imaging direction 111 of the OCT imaging system 110, as process S72 of Figure 7.

In process S91 of Figure 9, the controller 115 identifies (or locates) a representation of an anatomical feature of the eye 140 within the calibration B-scan 800 for determining the orientation of the axis in the calibration B-scan 800 corresponding to the pupillary axis 153 of the eye 140, relative to the direction in the calibration B-scan corresponding to the axial imaging direction 111 of the OCT imaging system 110. For example, in the calibration B-scan 800 in Figure 8, representations of opposing portions 804, 805 of the scleral spur of the eye 140 are identified (as the line 806 joining the opposing portions 804, 805 of the scleral spur, which is expected to be parallel to the plane of the pupil of the eye 140, may be used to approximate the orientation of the pupillary axis 153 of the eye 140 in the calibration B-scan 800 relative to the frame of the calibration B-scan 800). However, other anatomical features which are suitable for this purpose may be identified, such as the pupil being identified in the calibration B-scan 800 to obtain the plane of the pupil directly. Such representations of the scleral spur or other anatomical features may be identified using any of the techniques well-known to those skilled in the art. For example, the controller 115 may employ deep learning methods, which make use of trainable, multilayer neural networks, to identify the representations of the anatomical features in the calibration B-scan 800. As another example, the controller 115 may process the calibration B-scan 800 by causing it to be displayed to the user on a display screen, and acquire the identification of the representations of the anatomical features in the calibration B-scan 800 by receiving designations of these locations that have been input by the user (using a mouse and/or keyboard, or via one or more touch interactions in case the display screen is a touch-screen, for example) after having inspected the displayed calibration B-scan 800.

In process S92 of Figure 9, the controller 115 determines the orientation of the aforementioned axis in the calibration B-scan relative to the aforementioned direction in the calibration B-scan based on the identified representation of the anatomical feature of the eye 140. Continuing the example in the calibration B-scan 800 of Figure 8, a direction orthogonal to the line 806 in the plane of the calibration B-scan 800 is indicative of the direction of the axis 803 in the calibration B-scan 800. Thus, this orthogonal direction may be used to determine the orientation of the axis 803 in the calibration B-scan 800 relative to the direction 801 in the calibration B-scan 800.

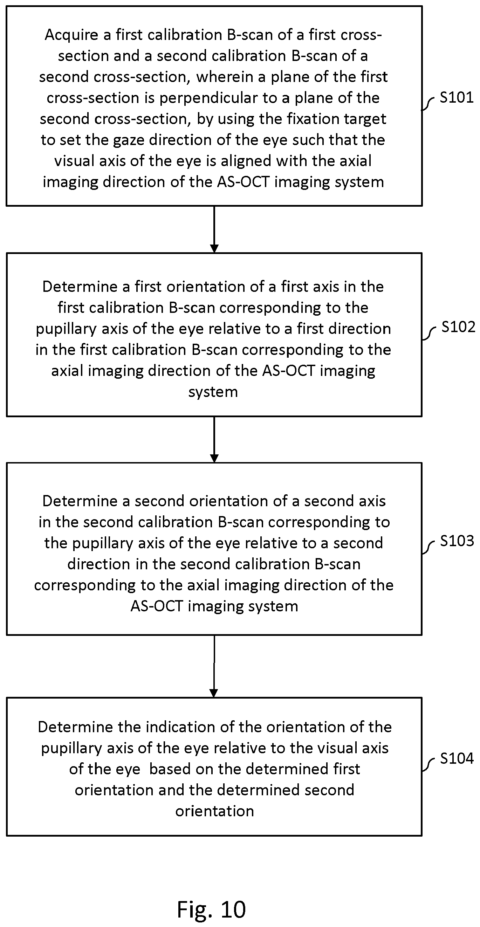

Figure 10 is a flow diagram illustrating a second alternative process by which the controller 115 of the OCT imaging system 110 may, as in a second alternative implementation of the present example embodiment, acquire the indication, *I*, of an orientation of the pupillary axis 153 of the eye 140 relative to the visual axis 152 of the eye 140. The second alternative process is similar to the first alternative process but acquires a three-dimensional ordination of the pupillary axis 153 of the eye 140 relative to the visual axis of the eye 140 thus making it particularly, but not exclusively, suited to aligning the pupillary axis 153 of the eye 140 with the axial imaging direction 111 of the OCT imaging system 110 in C-scans (as the AS-OCT image 130). Where the second alternative process described below is used, process S61 in Figure 6 may be omitted.

In process S101 of Figure 10, the controller 115 controls the OCT imaging system 110 to acquire a first calibration B-scan of a first cross-section of at least a portion of the anterior segment of the eye 140, for determining a first orientation of a first axis in the first calibration B-scan corresponding to the pupillary axis 153 of the eye 140 relative to a first direction in the first calibration B-scan corresponding to the axial imaging direction 111 of the OCT imaging system 110 (e.g. by the first calibration B-scan having the first axis corresponding to the pupillary axis 153 of the eye 140). In process S101 of Figure 10, the controller 115 further controls the OCT imaging system 110 to acquire a second calibration B-scan of a second cross-section of at least a portion of the anterior segment of the eye 140, for determining a second orientation of a second axis in the second calibration B-scan corresponding to the pupillary axis 153 of the eye 140 relative to a second direction in the second calibration B-scan corresponding to the axial imaging direction 111 of the OCT imaging system 110 (e.g. by the second calibration B-scan having the second axis corresponding to the pupillary axis 153 of the eye 140). Here, a plane of the first cross-section is perpendicular to a plane of the second cross-section. The controller 115 does this by controlling the fixation target 120 to set the gaze direction 151 of the eye 140 such that the visual axis 152 of the eye 140 is aligned with the axial imaging direction 111 of the OCT imaging system 110. That is, during the acquisition of the first and second calibration B-scans, the controller 115 sets a position of the fixation target 110 relative to the eye 140 such that, when the gaze direction of the eye 140 is fixed by the fixation target 120 at the set position, the gaze direction 151 of the eye 140 (along which the visual axis of the eye 140 runs) is aligned with the axial imaging direction 111 of the OCT imaging system 110. The first cross-section and the second cross-section may each be of the whole anterior chamber of the eye 140 and surrounding portions of the anterior segment of the eye 140, and each may include at least one portion of the scleral spur of the eye 140.

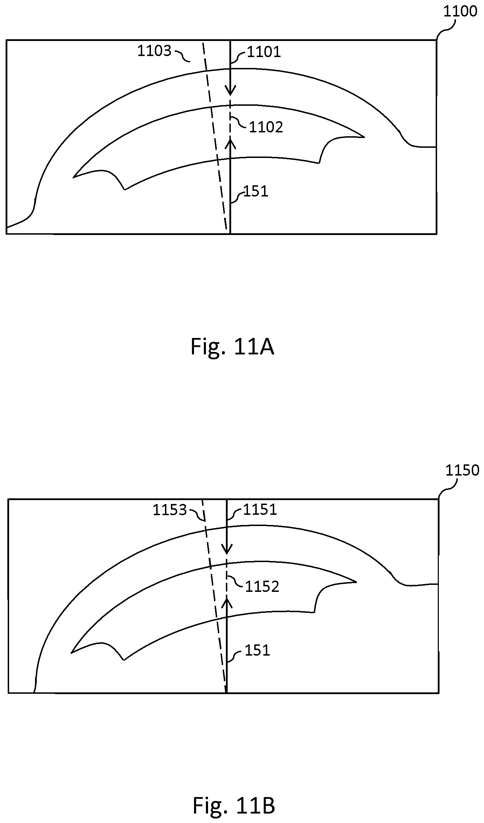

Figures 11A and 11B are schematic illustrations of an example first calibration B-scan 1100 and an example second calibration B-scan 1150, respectively. As shown, respective axes 1102, 1152 in the calibration B-scans 1100, 1150 corresponding to the visual axis of the eye 140 are parallel with respective directions 1101, 1151 in the calibration B-scans 1100, 1150 corresponding to the axial imaging direction 111 of the OCT imaging system 110, although they may, more generally, be aligned with their respective directions 1101, 1151 within a predetermined tolerance (e.g. 0.01, 0.1 or 1 degrees) from their respective directions 1101, 1151.

In process S102 of Figure 10, the controller 115 determines the first orientation of the first axis in the first calibration B-scan corresponding to the pupillary axis 153 of the eye 140 relative to the first direction in the first calibration B-scan corresponding to the axial imaging direction 111 of the OCT imaging system 110. For example, in Figure 11A the OCT imaging system 110 determines the first orientation as the orientation of the axis 1103 corresponding to pupillary axis 153 of the eye 140 relative to the direction 1101 in the first calibration B-scan 1100 corresponding to the axial imaging direction 111 of the OCT imaging system 110. The controller 115 may determine the first orientation via the process of Figure 9, as described in detail above.

In process S103 of Figure 10, the controller 115 determines the second orientation of the second axis in the second calibration B-scan corresponding to the pupillary axis 153 of the eye 140 relative to the second direction in the second calibration B-scan corresponding to the axial imaging direction 111 of the OCT imaging system 110. For example, with reference to Figure 11B, the controller 115 determines the second orientation as the orientation of the axis 1153 corresponding to pupillary axis 153 of the eye 140 relative to the direction 1151 in the second calibration B-scan 1050 corresponding to the axial imaging direction 111 of the OCT imaging system 110. The controller 115 may determine the second orientation via the process of Figure 9, as described above.

In process S104 of Figure 10, the controller 115 determines the indication, *I*, of the orientation of the pupillary axis 153 of the eye 140 relative to the visual axis 152 of the eye 140 based on the first orientation (determined in process S102) and the second orientation (determined in process S103). As the first cross-section is perpendicular to the second cross-section, the determined first orientation and the determined second orientation define the orientation of the pupillary axis 153 of the eye 140 relative to the visual axis 152 of the eye 140 in three dimensions relative to the frame of the first cross-section and/or second cross-section. Accordingly, the controller 115 may use the determined first orientation and the determined second orientation as the indication, *I*, of the orientation of the pupillary axis 153 of the eye 140 relative to the visual axis 152 of the eye 140, or may resolve the determined first orientation and the determined second orientation into a first component in the nasal-inferior direction (using the acquired indication of the laterality of the eye 140 or, where no indication is acquired, resolve the first component relative to a direction along horizontal axis falling along the nasal inferior direction) and a second component in the superior-inferior direction as the indication, *I*, of the orientation of the pupillary axis 153 of the eye 140 relative to the visual axis 152 of the eye 140, for example. Alternatively, where the AS-OCT image 130 is the B-scan 200, the OCT imaging system 110 may determine the indication, *I*, of the orientation of the pupillary axis 153 of the eye 140 relative to the visual axis 152 of the eye 140 based on the determined first orientation and the determined second orientation by resolving the determined first orientation and the determined second orientation into the plane of the cross-section of B-scan 200 and using this as the determined indication, *I*.

The first alternative process and the second alternative process described above may allow a patient-specific alignment of the pupillary axis 153 of the eye 140 to the axial imaging direction 111 of the OCT imaging system 110 to be performed, further improving the accuracy and reliability of the acquired geometric measurements of the eye 140 due to the difference in image contrast between representations of opposing portions of the scleral spur of the eye in the AS-OCT image 130 being further reduced. However, the process in the first example embodiment which makes use of measurements of the relative orientations from a sample set of eyes may allow faster alignment to be achieved, without the need to acquire one or more calibration AS-OCT images, thus reducing the acquisition time of the geometric measurements of the eye 140 as compared to the alternative implementations. This process may therefore be preferable for clinical applications, where process efficiency/throughput is important and the achievable improvement in accuracy of the acquired geometric measurements of the eye 140 may be sufficient to not warrant the use of the alternative implementations described above.

Returning again to Figure 6, in process S63, the controller 115 uses the acquired indication, *I*, of the orientation of the pupillary axis 153 of the eye 140 relative to the visual axis 152 of the eye 140 to set the fixation target 120 so that it fixes the gaze direction 151 of the eye 140 during acquisition of the AS-OCT image 130 such that the axis 203 in the AS-OCT image 130 is aligned with the direction 201 in the AS-OCT image 130. The OCT imaging system 110 may, as in the present example embodiment, achieve this by setting the position of the fixation target 120 using the acquired indication, *I*, of the orientation of the pupillary axis 153 of the eye 140 relative to the visual axis 152 of the eye 140 such that the gaze direction 151 of the eye 140 is aligned, within a predetermined tolerance (e.g. 0.01, 0.1 or 1 degrees), with the axial imaging direction 111 of the OCT imaging system 110. Thus, the B-scan 200 of Figure 2 may be acquired by the OCT imaging system 110 in any cross-sectional plane of the eye 140 and have the axis 203 parallel to the direction 201. Similarly, where the AS-OCT image 130 is provided in the form of an OCT C-scan, as described above, the alignment of the gaze direction 151 of the eye 140 with the axial imaging direction 111 in three-dimensional space may result in an axis in the C-scan corresponding to the pupillary axis of the eye 140 being aligned with a direction in the C-scan corresponding to the axial imaging direction 111.

However, the controller 115 may alternatively achieve this by setting the position of the fixation target 120 using the acquired indication, *I*, of the orientation of the pupillary axis 153 of the eye 140 relative to the visual axis 152 of the eye 140 such that a projection of the gaze direction 151 of the eye 140 into a plane is aligned with a projection of the axial imaging direction 111 of the OCT imaging system 110 onto the plane, the plane being the plane in which the B-scan 200 is acquired. For example, the acquired indication, *I*, of the orientation in process S52 (or the alternative implementations thereof) may provide information for setting the position of the fixation target 120 such that the axis 203 in the B-scan 200 is aligned with the direction 201 in the B-scan 200, by the controller 115 having already resolved the acquired indication in process S62 or 5104 into the (anticipated or predetermined) plane of the B-scan 200 or having acquired the indication from a calibration B-scan parallel to the B-scan 200 in process S72. The controller 115 may alternatively perform such resolving of the acquired indication at this stage. For example, where the acquired indication is of an orientation with components in two directions, the controller 115 may use information on the position of the plane of the B-scan 200 relative to, for example, the nasal-temporal and the superior-inferior directions to resolve the acquired indication, *I*, of the orientation of the pupillary axis 153 of the eye 140 relative to the visual axis 152 of the eye 140 into the plane of the B-scan 200 at this stage to obtain an orientation Ø*_{b}* of the pupillary axis 153 of the eye 140 relative to the visual axis 152 of the eye 140 as it will appear in the B-scan 200. Thus, the controller 115 may set the position of the fixation target 120 using the orientation Ø*_{b}* such that the axis 203 in the B-scan 200 is parallel with the direction 201 in the B-scan 200.

Alternatively, where the acquired indication, *I*, in process S62 is an orientation in one direction (e.g. in the nasal-temporal or the superior-inferior direction), the controller 115 may set the position of the fixation target 120 such that, if the B-scan 200 or future B-scans are acquired in the plane within which the acquired orientation is defined in the one direction, the axis in the B-scan corresponding to the pupillary axis of the eye 140 will be aligned with the direction in the B-scan corresponding to the axial imaging direction 110 of the OCT imaging system 110.

Although the fixation target 120 is described above as being arranged to fix the gaze direction 151 of the eye 140 during acquisition of the AS-OCT image 130 such that the axis 203 in the AS-OCT image 130 corresponding to a pupillary axis 153 of the eye 140 is aligned with a direction 201 in the AS-OCT image 130 corresponding to an axial imaging direction 111 of the OCT imaging system 110, the fixation target 120 may alternatively be arranged to fix the gaze direction 151 of the eye 140 during acquisition of the AS-OCT image 130 such that an axis in the AS-OCT image 130 corresponding to an optical axis of the eye 140 is aligned with the direction in the AS-OCT image 130 corresponding to the axial imaging direction 111 of the OCT imaging system 110. In such a case, in process S62 of Figure 6, the measurements instead relate to the relative orientations of the optical axis and the visual axis in eyes of a sample set of eyes (i.e., the alpha angle of eyes in the sample set of eyes, rather than the kappa angle) and in the alternate processes in Figure 7 and Figure 10, the respective orientations in the respective calibration B-scans are of a respective axis in the respective calibration B-scan corresponding to the optical axis of the eye 140 relative to a respective direction in the respective B-scan corresponding to the axial imaging direction 111 of the OCT imaging system 110. More generally, the fixation target 120 may be arranged to fix the gaze direction 151 of the eye 140 during acquisition of the AS-OCT image 130 such that the locations of the representations of opposing portions of the scleral spur of the eye 140 in the AS-OCT image 130 in the direction in the AS-OCT image 130 corresponding to the axial imaging direction 111 of the OCT imaging system 110 (i.e. the axial direction in the AS-OCT image 130, along which data elements of the component A-scans are arrayed) are level with respect to the direction. For example, in the case of the AS-OCT image 130 being a B-scan, the representations of opposing portions of the scleral spur would appear at the same height in the B-scan. That is, the locations of the representations of opposing portions of the scleral spur of the eye 140 in the AS-OCT image 130 in the axial direction in the AS-OCT image 130 are equal (within a predefined tolerance) along that direction. In other words, the fixation target 120 may be arranged to fix the gaze direction 151 of the eye 140 during acquisition of the AS-OCT image such that the representations of the opposing portions of the scleral spur of the eye 140 in the AS-OCT image 130 have respective axial depths (i.e. respective locations along the axial direction in the AS-OCT image 130) that are substantially the same.

In the first example embodiment and its variants described above, the described control of the fixation target 120 can cause the image contrast of the representations of opposing portions of the scleral spur of the eye 140 in the AS-OCT image 130 acquired by the OCT imaging system 110 to be same or nearly the same. Accordingly, the requisite degree of alignment between the axis 203 in the AS-OCT image 130 corresponding to the pupillary axis 153 of the eye 140 and the direction 201 in the AS-OCT image 130 corresponding to the axial imaging direction 111 of the OCT imaging system 110 may be such that an acceptable degree of equalisation of the scleral spur contrasts can be achieved.

For example, Figure 12A shows a B-scan 1200 acquired by a conventional OCT imaging system, to which tilt correction would normally be applied in post-processing only. As can be seen in Fig. 12A, the difference in image contrast between the representations 1201 and 1202 of opposing portions of the scleral spur of the eye 140 is significant, which would limit the accuracy and reliability of geometric measurements of the eye determined based on the B-scan 1200. For comparison, Figure 12B shows a B-scan 1250 acquired by the OCT imaging system 110 according to the present example embodiment. As can be seen in Figure 12B, the contrast between the representations 1251 and 1252 of opposing portions of the scleral spur of the eye 140 is approximately equal, which may significantly improve the accuracy and reliability of the geometric measurements.

Some example embodiments described above are summarised in the following numbered clauses E1 to E14.

E1. The use of a fixation target 120 of an optical coherence tomography, OCT, imaging system to equalise image contrast in representations of opposing portions of a scleral spur of an eye 140 in an anterior-segment OCT, AS-OCT, image acquired by the OCT imaging system 110, wherein the fixation target 120 is used to equalise the image contrast by fixing a gaze direction 151 of the eye 140 during acquisition of the AS-OCT image 130 such that an axis in the AS-OCT image 130 corresponding to a pupillary axis 153 of the eye 140 is aligned with a direction in the AS-OCT image 130 corresponding to an axial imaging direction 111 of the OCT imaging system 110.

E2. The use according to E1, wherein the fixation target 120 is used to equalise the image contrast by fixing the gaze direction 151 of the eye 140 during acquisition of the AS-OCT image 130 such that the axis in the AS-OCT image 130 corresponding to the pupillary axis 153 of the eye 140 is aligned with the direction in the AS-OCT image 130 corresponding to the axial imaging direction 111 of the OCT imaging system 110 by:
acquiring an indication of a laterality of the eye 140;
acquiring an indication of an orientation of the pupillary axis 153 of the eye 140 relative to a visual axis of the eye 140, based on measurements of relative orientations of the pupillary axis and the visual axis in eyes of a sample set of eyes and the acquired indication of the laterality of the eye 140; and
using the acquired indication of the orientation of the pupillary axis 153 of the eye 140 relative to the visual axis of the eye 140 to set a position of the fixation target 120 relative to the eye 140 such that, when the gaze direction 151 of the eye 140 is fixed by the fixation target 120 at the set position, the axis in the AS-OCT image 130 is aligned with the direction in the AS-OCT image 130.

E3. The use according to E1, wherein
the AS-OCT image 130 is a B-scan 200 comprising the representations of the opposing portions of the scleral spur of the eye 140, and
the fixation target 120 is used to equalise the image contrast by fixing the gaze direction 151 of the eye 140 during acquisition of the AS-OCT image 130 such that the axis in the AS-OCT image 130 corresponding to the pupillary axis 153 of the eye 140 is aligned with the direction in the AS-OCT image 130 corresponding to the axial imaging direction 111 of the OCT imaging system 110 by:
   acquiring an indication of an orientation of the pupillary axis 153 of the eye 140 relative to a visual axis of the eye 140 by:
      acquiring a calibration B-scan 800 of at least a portion of the anterior segment of the eye 140, wherein the calibration B-scan 800 is parallel to the B-scan 200, by using the fixation target 120 to set the gaze direction 151 of the eye 140 such that the visual axis of the eye 140 is aligned with the axial imaging direction 111 of the OCT imaging system 110; and
      determining, as the indication of the orientation of the pupillary axis 153 of the eye 140 relative to the visual axis of the eye 140, an orientation of an axis 803 in the calibration B-scan 800 corresponding to the pupillary axis 153 of the eye 140 relative to a direction 801 in the calibration B-scan 800 corresponding to the axial imaging direction 111 of the OCT imaging system 110; and
   using the acquired indication of the orientation to set a position of the fixation target 120 relative to the eye 140 during acquisition of the AS-OCT image 130 such that, when the gaze direction 151 of the eye 140 is fixed by the fixation target 120 at the set position, the axis 203 in the B-scan 200 is aligned with the direction 201 in the B-scan 200.

E4. The use according to E3, wherein the orientation of the axis 803 in the calibration B-scan 800 corresponding to the pupillary axis 153 of the eye 140 relative to the direction 801 in the calibration B-scan 800 corresponding to the axial imaging direction 111 of the OCT imaging system 110 is determined by:
identifying a representation of an anatomical feature 804, 805 of the eye 140 within the calibration B-scan 800 for determining the orientation of the axis 803 in the calibration B-scan 800 relative to the direction 801 in the calibration B-scan 800; and
determining the orientation of the axis 803 in the calibration B-scan 800 relative to the direction 801 in the calibration B-scan 800 based on the identified representation of the anatomical feature 804, 805 of the eye 140.

E5. The use according to E1, wherein the fixation target 120 is used to equalise the image contrast by fixing the gaze direction 151 of the eye 140 during acquisition of the AS-OCT image 130 such that the axis in the AS-OCT image 130 corresponding to the pupillary axis 153 of the eye 140 is aligned with the direction in the AS-OCT image 130 corresponding to the axial imaging direction 111 of the OCT imaging system 110 by:
acquiring an indication of an orientation of the pupillary axis 153 of the eye 140 relative to a visual axis of the eye 140 by:
   acquiring a first calibration B-scan 1100 of a first cross-section of at least a portion of the anterior segment of the eye 140, and a second calibration B-scan 1140 of a second cross-section of at least a portion of the anterior segment of the eye 140, wherein a plane of the first cross-section is perpendicular to a plane of the second cross-section, by using the fixation target 120 to set the gaze direction 151 of the eye 140 such that the visual axis of the eye 140 is aligned with the axial imaging direction 111 of the OCT imaging system 110;
   determining a first orientation of a first axis 1103 in the first calibration B-scan 1100 corresponding to the pupillary axis 153 of the eye 140 relative to a first direction 1101 in the first calibration B-scan 1100 corresponding to the axial imaging direction 111 of the OCT imaging system 110;
   determining a second orientation of a second axis 1153 in the second calibration B-scan 1140 corresponding to the pupillary axis 153 of the eye 140 relative to a second direction 151 in the second calibration B-scan 1140 corresponding to the axial imaging direction 111 of the OCT imaging system 110; and
   determining the indication of the orientation of the pupillary axis 153 of the eye 140 relative to the visual axis of the eye 140 based on the determined first orientation and the determined second orientation; and
using the acquired indication of the orientation to set a position of the fixation target 120 relative to the eye 140 during acquisition of the AS-OCT image 130 such that, when the gaze direction 151 of the eye 140 is fixed by the fixation target 120 at the set position, the axis in the AS-OCT image 130 corresponding to the pupillary axis 153 of the eye 140 is aligned with the direction in the AS-OCT image 130 corresponding to the axial imaging direction 111 of the OCT imaging system 110.

E6. The use according to E5, wherein
the first orientation is determined by:
   identifying a representation of a first anatomical feature of the eye 140 within the first calibration B-scan 1100 for determining the orientation of the first axis 1103 in the first calibration B-scan 1100 corresponding to the pupillary axis 153 of the eye 140 relative to the first direction 1101 in the first calibration B-scan 1100 corresponding to the axial imaging direction 111 of the OCT imaging system 110; and
   determining the orientation of the first axis 1103 in the first calibration B-scan 1100 corresponding to the pupillary axis 153 of the eye 140 relative to the first direction 1101 in the first calibration B-scan 1100 corresponding to the axial imaging direction 111 of the OCT imaging system 110 based on the representation of the first anatomical feature of the eye 140 within the first calibration B-scan 1100,
and the second orientation is determined by:
   identifying a representation of a second anatomical feature of the eye 140 within the second calibration B-scan 1140 for determining the orientation of the second axis 1153 in the second calibration B-scan 1140 corresponding to the pupillary axis 153 of the eye 140 relative to the second direction 1151 in the second calibration B-scan 1140 corresponding to the axial imaging direction 111 of the OCT imaging system 110; and
   determining the orientation of the second axis 1153 in the second calibration B-scan 1140 corresponding to the pupillary axis 153 of the eye 140 relative to the second direction 1151 in the second calibration B-scan 1140 corresponding to the axial imaging direction 111 of the OCT imaging system 110 based on the representation of the second anatomical feature of the eye 140 within the second calibration B-scan 1140.

E7. The use according to any one of E1 to E6, wherein the fixation target 120 comprises a graphic 310 which is displayed by a display device 300, wherein a display position of the graphic 310 relative to the eye 140 is controllable so as to control the gaze direction 151 of the eye 140 when the eye 140 fixates on the graphic 310.

E8. A method of processing an anterior-segment optical coherence tomography, AS-OCT, image acquired by an OCT imaging system 110, which comprises representations of opposing portions of a scleral spur of an eye, to obtain a geometric measurement 160 based on one or more anatomical features in the AS-OCT image 130, the method comprising:
acquiring S51 the AS-OCT image 130 whilst a gaze direction 151 of the eye 140 is fixed by the eye 140 fixating on a fixation target 120 such that an axis in the AS-OCT image 130 corresponding to a pupillary axis 153 of the eye 140 is aligned with a direction in the AS-OCT image 130 corresponding to an axial imaging direction 111 of the OCT imaging system 110;
processing S52 the acquired AS-OCT image 130 to acquire respective locations in the AS-OCT image 130 of the representations of the opposing portions of the scleral spur of the eye 140 in the AS-OCT image 130; and
obtaining S53 the geometric measurement 160 based on the acquired locations in the AS-OCT image 130.

E9. The method according to E8, further comprising setting the fixation target 120 to fix the gaze direction 151 of the eye 140 during acquisition of the AS-OCT image 130 by:
acquiring S61 an indication of a laterality of the eye 140;
acquiring S62 an indication of an orientation of the pupillary axis 153 of the eye 140 relative to a visual axis of the eye 140, based on measurements of relative orientations of the pupillary axis and the visual axis in eyes of a sample set of eyes and the acquired indication of the laterality of the eye 140; and
using S63 the acquired indication of the orientation of the pupillary axis 153 of the eye 140 relative to the visual axis of the eye 140 to set a position of the fixation target 120 relative to the eye 140 such that, when the gaze direction 151 of the eye 140 is fixed by the fixation target 120 at the set position, the axis in the AS-OCT image 130 is aligned with the direction in the AS-OCT image 130.

E10. The method according to E8, wherein the AS-OCT image 130 is a B-scan 200 comprising the representations of the opposing portions of the scleral spur of the eye, and the fixation target 120 is set to fix the gaze direction 151 of the eye 140 during acquisition of the B-scan 200 by:
acquiring an indication of an orientation of the pupillary axis 153 of the eye 140 relative to a visual axis of the eye 140 by:
   acquiring S71 a calibration B-scan 800 of at least a portion of anterior segment of the eye 140, wherein the calibration B-scan 800 is parallel to the B-scan 200, by using the fixation target 120 to set the gaze direction 151 of the eye 140 such that the visual axis of the eye 140 is aligned with the axial imaging direction 111 of the OCT imaging system 110; and
   determining S72, as the indication of the orientation of the pupillary axis 153 of the eye 140 relative to the visual axis of the eye 140, an orientation of an axis 803 in the calibration B-scan 800 corresponding to the pupillary axis 153 of the eye 140 relative to a direction 801 in the calibration B-scan 800 corresponding to the axial imaging direction 111 of the OCT imaging system 110; and
using S63 the acquired indication of the orientation to set a position of the fixation target 120 relative to the eye 140 during acquisition of the AS-OCT image 130 such that, when the gaze direction 151 of the eye 140 is fixed by the fixation target 120 at the set position, the axis in the B-scan is aligned with the direction in the B-scan.

E11. The method according to E10, wherein the orientation of the axis 803 in the calibration B-scan 800 relative to the direction 801 in the calibration B-scan 800 is determined by:
identifying S91 a representation of an anatomical feature 804, 805 of the eye 140 within the calibration B-scan 800 for determining the orientation of the axis 803 in the calibration B-scan 800 relative to the direction 801 in the calibration B-scan 800; and
determining S92 the orientation of the axis 803 in the calibration B-scan 800 relative to the direction 801 in the calibration B-scan 800 based on the identified representation of the anatomical feature 804, 805 of the eye 140.

E12. The method according to E8, wherein the fixation target 120 is set to fix the gaze direction 151 of the eye 140 during acquisition of the B-scan 200 by:
acquiring an indication of an orientation of the pupillary axis 153 of the eye 140 relative to a visual axis of the eye 140 by:
acquiring S101 a first calibration B-scan 1100 of a first cross-section of at least a portion of the anterior segment of the eye 140 , and a second calibration B-scan 1140 of a second cross-section of at least a portion of the anterior segment of the eye 140, wherein a plane of the first cross-section is perpendicular to a plane of the second cross-section, the first calibration B-scan 1100 and the second calibration B-scan 1140 being acquired using the fixation target 120 to set the gaze direction 151 of the eye 140 such that the visual axis of the eye 140 is aligned with the axial imaging direction 111 of the OCT imaging system 110;
determining S102 a first orientation of a first axis 1103 in the first calibration B-scan 1100 corresponding to the pupillary axis 153 of the eye 140 relative to a first direction 1101 in the first calibration B-scan 1100 corresponding to the axial imaging direction 111 of the OCT imaging system 110;
determining S103 a second orientation of a second axis 1153 in the second calibration B-scan 1140 corresponding to the pupillary axis 153 of the eye 140 relative to a second direction 1151 in the second calibration B-scan 1140 corresponding to the axial imaging direction 111 of the OCT imaging system 110; and
determining S104 the indication of the orientation of the pupillary axis 153 of the eye 140 relative to the visual axis of the eye 140 based on the determined first orientation and the determined second orientation; and
using S63 the acquired indication of the orientation to set a position of the fixation target 120 relative to the eye 140 during acquisition of the AS-OCT image 130 such that, when the gaze direction 151 of the eye 140 is fixed by the fixation target 120 at the set position, the axis in the AS-OCT image 130 corresponding to the pupillary axis 153 of the eye 140 is aligned with the direction in the AS-OCT image 130 corresponding to the axial imaging direction 111 of the OCT imaging system 110.

E13. The method according to E12, wherein
the orientation of the first axis 1103 in the first calibration B-scan 1100 relative to the first direction 1101 in the first calibration B-scan 1100 is determined by:
   identifying S91 a representation of a first anatomical feature of the eye 140 within the first calibration B-scan 1100 for determining the orientation of the first axis 1103 in the first calibration B-scan 1100 corresponding to the pupillary axis 153 of the eye 140 relative to the first direction 1101 in the first calibration B-scan 1100 corresponding to the axial imaging direction 111 of the OCT imaging system 110; and
   determining S91 the orientation of the first axis 1101 in the first calibration B-scan 1100 corresponding to the pupillary axis 153 of the eye 140 relative to the first direction 1103 in the first calibration B-scan 1100 corresponding to the axial imaging direction 111 of the OCT imaging system 110 based on the representation of the first anatomical feature of the eye 140 within the first calibration B-scan 1100, and
the orientation of the second axis 1153 in the second calibration B-scan 1140 relative to the second direction 1151 in the second calibration B-scan 1140 is determined by:
   identifying S91 a representation of a second anatomical feature of the eye 140 within the second calibration B-scan 1140 for determining the orientation of the second axis 1153 in the second calibration B-scan 1140 corresponding to the pupillary axis 153 of the eye 140 relative to the second direction 1151 in the second calibration B-scan 1140 corresponding to the axial imaging direction 111 of the OCT imaging system 110; and
   determining S92 the orientation of the second axis 1153 in the second calibration B-scan 1140 corresponding to the pupillary axis 153 of the eye 140 relative to the second direction 1151 in the second calibration B-scan 1140 corresponding to the axial imaging direction 111 of the OCT imaging system 110 based on the representation of the second anatomical feature of the eye 140 within the second calibration B-scan 1140.

E14. The method according to any of E8 to E13, wherein the geometric measurement 160 is a measurement of an anterior chamber angle of the eye 140.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the functions of the controller 115 and/or the data processing hardware 140, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, floppy diskettes, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some or all of the functionality of the controller 115 and/or the data processing hardware 140may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. For example, while the processes shown in Figures 5 to 7, 9 and 10 are performed in the order indicated by arrows between the steps therein, some of the processes may be executed in parallel. For example, processes S102 and S103 in Figure 10 may be performed in parallel. Thus, the present invention should not be limited by any of the above-described example embodiments, but should be defined only in accordance with the following claims and their equivalents.

Further, the purpose of the Abstract is to enable the Patent Office and the public generally, and especially the scientists, engineers and practitioners in the art who are not familiar with patent or legal terms or phraseology, to determine quickly from a cursory inspection the nature and essence of the technical disclosure of the application. The Abstract is not intended to be limiting as to the scope of the example embodiments presented herein in any way. It is also to be understood that any procedures recited in the claims need not be performed in the order presented.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. A system (100) arranged to process an anterior-segment optical coherence tomography, AS-OCT, image (130) comprising representations of portions of a scleral spur of an eye (140) to obtain a geometric measurement (160) of the eye (140), the system (100) comprising:
data processing hardware (140) arranged to:
process the AS-OCT image (130) to acquire respective locations in the AS-OCT image (130) of the representations of the portions of the scleral spur of the eye (140) in the AS-OCT image (130); and
obtain the geometric measurement (160) based on the acquired locations; and
an OCT imaging system (110) operable to acquire the AS-OCT image (130), the OCT imaging system (110) comprising a fixation target (120) arranged to fix a gaze direction (151) of the eye (140) during acquisition of the AS-OCT image (130) such that an axis in the AS-OCT image (130) corresponding to a pupillary axis (153) of the eye (140) is aligned with a direction in the AS-OCT image (130) corresponding to an axial imaging direction (111) of the OCT imaging system (110).

2. The system (100) according to Claim 1, wherein the OCT imaging system (110) comprises a controller (115) arranged to control the OCT imaging system (110) to acquire the AS-OCT image (130), the controller (115) being further arranged to control the fixation target (120) to fix the gaze direction (151) of the eye (140) during acquisition of the AS-OCT image (130) by:
acquiring an indication of a laterality of the eye (140);
acquiring an indication of an orientation of the pupillary axis (153) of the eye (140) relative to a visual axis of the eye (140), based on measurements of relative orientations of the pupillary axis and the visual axis in eyes of a sample set of eyes and the acquired indication of the laterality of the eye (140); and
using the acquired indication of the orientation of the pupillary axis (153) of the eye (140) relative to the visual axis of the eye (140) to set a position of the fixation target (120) relative to the eye (140) such that, when the gaze direction (151) of the eye (140) is fixed by the fixation target (120) at the set position, the axis in the AS-OCT image (130) is aligned with the direction in the AS-OCT image (130).

3. The system (100) according to Claim 1, wherein the AS-OCT image (130) is a B-scan (200) comprising the representations of the portions of the scleral spur of the eye (140), and the OCT imaging system (110) comprises a controller (115) arranged to control the OCT imaging system (110) to acquire the AS-OCT image (130), the controller (115) being further arranged to control the fixation target (120) to fix the gaze direction (151) of the eye (140) during acquisition of the B-scan (200) by:
acquiring an indication of an orientation of the pupillary axis (153) of the eye (140) relative to a visual axis of the eye (140) by:
controlling the OCT imaging system (110) to acquire a calibration B-scan (800) of at least a portion of the anterior segment of the eye (140), wherein the calibration B-scan (800) is parallel to the B-scan, by controlling the fixation target (120) to set the gaze direction (151) of the eye (140) such that the visual axis of the eye (140) is aligned with the axial imaging direction (111) of the OCT imaging system (110); and
determining, as the indication of the orientation of the pupillary axis (153) of the eye (140) relative to the visual axis of the eye (140), an orientation of an axis (803) in the calibration B-scan (800) corresponding to the pupillary axis (153) of the eye (140) relative to a direction (801) in the calibration B-scan (800) corresponding to the axial imaging direction (111) of the OCT imaging system (110); and
using the acquired indication of the orientation to set a position of the fixation target (120) relative to the eye (140) during acquisition of the AS-OCT image (130) such that, when the gaze direction (151) of the eye (140) is fixed by the fixation target (120) at the set position, the axis (203) in the B-scan (200) is aligned with the direction (201) in the B-scan (200).

4. The system (100) according to Claim 3, wherein the controller (115) is arranged to determine the orientation of the axis (803) in the calibration B-scan (800) relative to the direction (801) in the calibration B-scan (800) by:
identifying a representation of an anatomical feature (804, 805) of the eye (140) within the calibration B-scan (800) for determining the orientation of the axis (803) in the calibration B-scan (800) relative to the direction (801) in the calibration B-scan (800); and
determining the orientation of the axis (803) in the calibration B-scan (800) relative to the direction (801) in the calibration B-scan (800) based on the identified representation of the anatomical feature (804, 805) of the eye (140).

5. The system (100) according to Claim 1, wherein the OCT imaging system (110) comprises a controller (115) arranged to control the OCT imaging system (110) to acquire the AS-OCT image (130), the controller (115) being further arranged to control the fixation target (120) to fix the gaze direction (151) of the eye (140) during acquisition of the B-scan (200) by:
acquiring an indication of an orientation of the pupillary axis of the eye (140) relative to a visual axis of the eye (140) by:
controlling the OCT imaging system (110) to acquire a first calibration B-scan (1100) of a first cross-section of at least a portion of the anterior segment of the eye (140), and a second calibration B-scan (1140) of a second cross-section of at least a portion of the anterior segment of the eye (140), wherein a plane of the first cross-section is perpendicular to a plane of the second cross-section, by using the fixation target (120) to set the gaze direction (151) of the eye (140) such that the visual axis of the eye (140) is aligned with the axial imaging direction (111) of the OCT imaging system (110);
determining a first orientation of a first axis (1103) in the first calibration B-scan (1100) corresponding to the pupillary axis (153) of the eye (140) relative to a first direction (1101) in the first calibration B-scan (1100) corresponding to the axial imaging direction (111) of the OCT imaging system (110);
determining a second orientation of a second axis (1153) in the second calibration B-scan (1140) corresponding to the pupillary axis (153) of the eye (140) relative to a second direction (1151) in the second calibration B-scan (1140) corresponding to the axial imaging direction (111) of the OCT imaging system (110); and
determining the indication of the orientation of the pupillary axis (153) of the eye (140) relative to the visual axis of the eye (140) based on the determined first orientation and the determined second orientation; and using the acquired indication of the orientation to set a position of the fixation target (120) relative to the eye (140) during acquisition of the AS-OCT image (130) such that, when the gaze direction (151) of the eye (140) is fixed by the fixation target (120) at the set position, the axis in the AS-OCT image (130) corresponding to the pupillary axis (153) of the eye (140) is aligned with the direction in the AS-OCT image (130) corresponding to the axial imaging direction (111) of the OCT imaging system (110)

6. The system (100) according to Claim 5, wherein
the controller (115) is arranged to determine the orientation of the first axis (1103) in the first calibration B-scan (1100) relative to the first direction (1101) in the first calibration B-scan (1100) by:
identifying a representation of a first anatomical feature of the eye (140) within the first calibration B-scan (1100) for determining the orientation of the first axis (1103) in the first calibration B-scan (1100) corresponding to the pupillary axis (153) of the eye (140) relative to the first direction (1101) in the first calibration B-scan (1100) corresponding to the axial imaging direction (111) of the OCT imaging system (110); and
determining the orientation of the first axis (1103) in the first calibration B-scan (1100) corresponding to the pupillary axis (153) of the eye (140) relative to the first direction (1101) in the first calibration B-scan (1100) corresponding to the axial imaging direction (111) of the OCT imaging system (110) based on the representation of the first anatomical feature of the eye (140) within the first calibration B-scan (1100),
and wherein the controller (115) is arranged to determine the orientation of the second axis (1153) in the second calibration B-scan (1140) relative to the second direction (1151) in the second calibration B-scan (1140) by:
identifying a representation of a second anatomical feature of the eye (140) within the second calibration B-scan (1140) for determining the orientation of the second axis (1153) in the second calibration B-scan (1140) corresponding to the pupillary axis (153) of the eye (140) relative to the second direction (1151) in the second calibration B-scan (1140) corresponding to the axial imaging direction (111) of the OCT imaging system (110); and
determining the orientation of the second axis (1153) in the second calibration B-scan (1140) corresponding to the pupillary axis (153) of the eye (140) relative to the second direction (1151) in the second calibration B-scan (1140) corresponding to the axial imaging direction (111) of the OCT imaging system (110) based on the representation of the second anatomical feature of the eye (140) within the second calibration B-scan (1140).

7. The system (100) according to any preceding claim, wherein the geometric measurement (160) is a measurement of an anterior chamber angle of the eye (140).

8. The system (100) according to any preceding claim, wherein the OCT imaging system (110) further comprises a display device (300) arranged to display a graphic (310) as the fixation target, wherein the controller (115) is arranged to control a display position of the graphic (310) relative to the eye (140) so as to control the gaze direction (151) of the eye (140) when the eye (140) fixates on the graphic (310).

9. The use of a fixation target (120) of an optical coherence tomography, OCT, imaging system to equalise image contrast in representations of portions of a scleral spur of an eye (140) in an anterior-segment OCT, AS-OCT, image acquired by the OCT imaging system (110), wherein the fixation target (120) is used to equalise the image contrast by fixing a gaze direction (151) of the eye (140) during acquisition of the AS-OCT image (130) such that an axis in the AS-OCT image (130) corresponding to a pupillary axis (153) of the eye (140) is aligned with a direction in the AS-OCT image (130) corresponding to an axial imaging direction (111) of the OCT imaging system (110).

10. The use according to Claim 9, wherein the fixation target (120) is used to equalise the image contrast by fixing the gaze direction (151) of the eye (140) during acquisition of the AS-OCT image (130) such that the axis in the AS-OCT image (130) corresponding to the pupillary axis (153) of the eye (140) is aligned with the direction in the AS-OCT image (130) corresponding to the axial imaging direction (111) of the OCT imaging system (110) by:
acquiring an indication of a laterality of the eye (140);
acquiring an indication of an orientation of the pupillary axis (153) of the eye (140) relative to a visual axis of the eye (140), based on measurements of relative orientations of the pupillary axis and the visual axis in eyes of a sample set of eyes and the acquired indication of the laterality of the eye (140); and
using the acquired indication of the orientation of the pupillary axis (153) of the eye (140) relative to the visual axis of the eye (140) to set a position of the fixation target (120) relative to the eye (140) such that, when the gaze direction (151) of the eye (140) is fixed by the fixation target (120) at the set position, the axis in the AS-OCT image (130) is aligned with the direction in the AS-OCT image (130).

11. The use according to Claim 9, wherein
the AS-OCT image (130) is a B-scan (200) comprising the representations of the portions of the scleral spur of the eye (140), and
the fixation target (120) is used to equalise the image contrast by fixing the gaze direction (151) of the eye (140) during acquisition of the AS-OCT image (130) such that the axis in the AS-OCT image (130) corresponding to the pupillary axis (153) of the eye (140) is aligned with the direction in the AS-OCT image (130) corresponding to the axial imaging direction (111) of the OCT imaging system (110) by:
acquiring an indication of an orientation of the pupillary axis (153) of the eye (140) relative to a visual axis of the eye (140) by:
acquiring a calibration B-scan (800) of at least a portion of the anterior segment of the eye (140), wherein the calibration B-scan (800) is parallel to the B-scan, by using the fixation target (120) to set the gaze direction (151) of the eye (140) such that the visual axis of the eye (140) is aligned with the axial imaging direction (111) of the OCT imaging system (110); and
determining, as the indication of the orientation of the pupillary axis (153) of the eye (140) relative to the visual axis of the eye (140), an orientation of an axis (803) in the calibration B-scan (800) corresponding to the pupillary axis (153) of the eye (140) relative to a direction (801) in the calibration B-scan (800) corresponding to the axial imaging direction (111) of the OCT imaging system (110); and
using the acquired indication of the orientation to set a position of the fixation target (120) relative to the eye (140) during acquisition of the AS-OCT image (130) such that, when the gaze direction (151) of the eye (140) is fixed by the fixation target (120) at the set position, the axis (203) in the B-scan (200) is aligned with the direction (201) in the B-scan (200).

12. The use according to Claim 9, wherein the fixation target (120) is used to equalise the image contrast by fixing the gaze direction (151) of the eye (140) during acquisition of the AS-OCT image (130) such that the axis in the AS-OCT image (130) corresponding to the pupillary axis (153) of the eye (140) is aligned with the direction in the AS-OCT image (130) corresponding to the axial imaging direction (111) of the OCT imaging system (110) by:
acquiring an indication of an orientation of the pupillary axis (153) of the eye (140) relative to a visual axis of the eye (140) by:
acquiring a first calibration B-scan (1100) of a first cross-section of at least a portion of the anterior segment of the eye (140), and a second calibration B-scan (1140) of a second cross-section of at least a portion of the anterior segment of the eye (140), wherein a plane of the first cross-section is perpendicular to a plane of the second cross-section, by using the fixation target (120) to set the gaze direction (151) of the eye (140) such that the visual axis of the eye (140) is aligned with the axial imaging direction (111) of the OCT imaging system (110);
determining a first orientation of a first axis (1103) in the first calibration B-scan (1100) corresponding to the pupillary axis (153) of the eye (140) relative to a first direction (1101) in the first calibration B-scan (1100) corresponding to the axial imaging direction (111) of the OCT imaging system (110);
determining a second orientation of a second axis (1153) in the second calibration B-scan (1140) corresponding to the pupillary axis (153) of the eye (140) relative to a second direction (1151) in the second calibration B-scan (1140) corresponding to the axial imaging direction (111) of the OCT imaging system (110); and
determining the indication of the orientation of the pupillary axis (153) of the eye (140) relative to the visual axis of the eye (140) based on the determined first orientation and the determined second orientation; and
using the acquired indication of the orientation to set a position of the fixation target (120) relative to the eye (140) during acquisition of the AS-OCT image (130) such that, when the gaze direction (151) of the eye (140) is fixed by the fixation target (120) at the set position, the axis in the AS-OCT image (130) corresponding to the pupillary axis (153) of the eye (140) is aligned with the direction in the AS-OCT image (130) corresponding to the axial imaging direction (111) of the OCT imaging system (110).

13. The use according to any one of Claims 9 to 12, wherein the fixation target (120) comprises a graphic (310) which is displayed by a display device (300), wherein a display position of the graphic (310) relative to the eye (140) is controllable so as to control the gaze direction (151) of the eye (140) when the eye (140) fixates on the graphic (310).

14. A method of processing an anterior-segment optical coherence tomography, AS-OCT, image acquired by an OCT imaging system (110), which comprises representations of portions of a scleral spur of an eye (140), to obtain a geometric measurement (160) based on one or more anatomical features in the AS-OCT image (130), the method comprising:
acquiring (S51) the AS-OCT image (130) whilst a gaze direction (151) of the eye (140) is fixed by the eye (140) fixating on a fixation target (120) such that an axis in the AS-OCT image (130) corresponding to a pupillary axis (153) of the eye (140) is aligned with a direction in the AS-OCT image 130 corresponding to an axial imaging direction (111) of the OCT imaging system (110);
processing (S52) the acquired AS-OCT image (130) to acquire respective locations in the AS-OCT image (130) of the representations of the portions of the scleral spur of the eye (140) in the AS-OCT image (130); and
obtaining (S53) the geometric measurement (160) based on the acquired locations in the AS-OCT image (130).

15. The method according to Claim 14, further comprising setting the fixation target (120) to fix the gaze direction (151) of the eye (140) during acquisition of the AS-OCT image (130) by:
acquiring (S61) an indication of a laterality of the eye (140);
acquiring (S62) an indication of an orientation of the pupillary axis (153) of the eye (140) relative to a visual axis of the eye (140), based on measurements of relative orientations of the pupillary axis and the visual axis in eyes of a sample set of eyes and the acquired indication of the laterality of the eye (140); and
using (S63) the acquired indication of the orientation of the pupillary axis (153) of the eye (140) relative to the visual axis of the eye (140) to set a position of the fixation target (120) relative to the eye (140) such that, when the gaze direction (151) of the eye (140) is fixed by the fixation target (120) at the set position, the axis in the AS-OCT image (130) is aligned with the direction in the AS-OCT image (130).
